Europäisches Patentamt

European Patent Office

Office européen des brevets

(11) Publication number: **0 225 150**
**A2**

## (12) EUROPEAN PATENT APPLICATION

(21) Application number: 86309167.4

(51) Int. Cl.⁴: **C 12 M 1/00**

(22) Date of filing: 25.11.86

(30) Priority: 26.11.85 TT 72

(43) Date of publication of application:
10.06.87 Bulletin 87/24

(84) Designated Contracting States:
AT BE CH DE ES FR GB GR IT LI LU NL SE

(71) Applicant: **De Lima, Daniel**
**26, Coblentz Avenue Cascade**
**Port of Spain (TT)**

(72) Inventor: **De Lima, Daniel**
**26, Coblentz Avenue Cascade**
**Port of Spain (TT)**

(74) Representative: **Wynne-Jones, John Vaughan et al**
**Wynne-Jones, Lainé & James 22, Rodney Road**
**Cheltenham Gloucestershire GL50 1JJ (GB)**

(54) **Biomass digester.**

(57) An air-tight containment structure for the digestion of biomass may be collapsed under atmospheric pressure by drawing a vacuum therein, thereby compressing the contained fiber matrix and enhancing the wetting of the biomass. Reagents may be added in quantities to wet only an initial upper layer, and then dispersed downwardly into a further layer by addition of a displacing liquid.

FIG. 1

**Description**

BIOMASS DIGESTER

FIELD OF INVENTION

This invention relates to a system for digesting cellulose-based materials in the form of biomass to produce improved animal feed and useful chemicals. More particularly it relates to a containment structure for enclosing and manipulating biomass during the digestion, drying and wetting process and for methods of utilizing that structure.

BACKGROUND TO THE INVENTION

Cellulose-based biomass may be hydrolyzed to sugars and the sugars may then be used for the production of yeasts. The yeasts can then be used as animal food stuffs or the sugars applied to produce useful chemicals such as ethanol and furfural.

According to one known process a weak acid solution is applied as a reagent to the biomass to extract hemi cellulose and improve the permeability of the cellulose. The hemi-cellulose may be further hydrolyzed to hexoses and pentoses. One of the costs in this, or any biomass digester system, is the amount of reagent solution employed.

Biomass is generally fibrous and of low density. It is necessary to contact the biomass with the activating solution in order to initiate the desired reaction. The high percentage of voids in biomass has previously required the use of large amounts of reagents to effect the desired reaction.

It is also known to apply a vacuum to material to be digested, prior to application of a reagent. This proceedure has, however, been directed to de-aerating the digestable material itself so as to facilitate more thorough penetration of the reagent into the substance of the biomass itself. The application of vacuum in the prior art has not been resorted to as a means for compaction of the digestable biomass or to a reduction of the quantity of reagent required.

This invention relates to a structure for containing biomass for digestion. More particularly it provides a means by which reduced quantities of reagents may be used to effect various stages of the digestion process, and by which valuable reagents may be recovered at the end of the process.

A further useful aspect of the invention is that it provides a means for controlling the yeast-reaction, and in some applications, arresting the decay of biomatter in the digester.

SUMMARY OF THE INVENTION

The containment structure of the invention used for biomass digestion is provided with a compressible, enveloping air-tight enclosure, having an upper cover, which may be in the form of a flexible sheet, which is adapted to be displaced downwardly into said structure. Within this enveloping enclosure may be placed biomass, with reagent-distribution and collection systems installed above and below the biomass. When it is desired to wet the biomass with reagent, the requisite reduced quantity of reagent is applied either from above from where it may fall by gravity, or from below. A vacuum is then drawn within the enveloping enclosure allowing the atmospheric air pressure to compact the biomass and reduce the percentage of voids. This allows for the wetting of more biomass with a given quantity of reagent. Alternately, less reagent may be used to effect this wetting process.

Once the reagent has been infiltrated into the biomass matrix, the vacuum may be released and the reaction allowed to proceed on the basis that the reagent has been distributed with improved efficiency. Where useful gases are generated, they may be drawn off through a gas-extraction system. Where liquid products are produced they may be drawn off by gravity accumulation and removal through the collection system. This process may be enhanced by reapplying the pressure created by drawing a vacuum, thereby recompressing the biomass.

By a further feature of the invention, a limited quantity of reagent may be applied to the upper surface of the biomass, in a quantity insufficient to wet more than an upper layer, even with vacuum compression. Then the digestion process may be allowed to proceed in the wetted upper layer.

Subsequently, when the digestion process has been essentially exhausted in the upper layer, a displacing liquid may be applied from the upper distribution system. This liquid is so selected as to cause the residual reagent to be moved downward into a lower layer within the bionmass, that has not been exhausted through digestion. Water can serve as a suitable liquid for this purpose, even allowing that it may partially enter into a solution with the reagent. This displacement process may again be facilitated by drawing a vacuum within the enveloping enclosure so as to allow the atmospheric pressure to compress the fiber matrix.

This process of displacing the active reagent downward may be repeated through successive layers, thus effecting a wave of zone reactions over time.

By a further feature of the invention the acidity of the wetted biomass in its sealed enclosure may be adjusted to such a degree e.g. 3.0 pH, that the microbial activity therein is arrested. The biomass in the digester may then be stored in this "pickled" condition pending a future date when it is desired for the reaction to continue. Reactivation of certain yeast growth is effected through raising the pH.

By a further feature of the invention the floor of the containment structure may be provided with localized inflatable bladders. By inflating such bladders with air or water the biomass matrix may be disturbed and agitated so as to enhance the efficiency of the digestion process. The bladders may be so placed between the inlets of the collection system so as to direct the flow of fluids under gravity towards such inlets.

These and other features of the invention will be apparent from the description of the preferred embodiment that follows hereafter.

## SUMMARY OF THE DRAWINGS

Figure 1 depicts in cross-section a biomass digester containment structure, according to the invention;

Figure 2 shows the containment structure of Figure 1 with a vacuum being drawn;

Figure 3 shows an overhead view of the collection system within the container of Figure 1;

Figure 4 shows the air tight connection between the upper and lower sheets that contain the biomass;

Figure 5 shows the biomass containment system in the course of depositing a limited quantity of reagent onto a localized upper layer;

Figure 6 shows the progressive displacement downwards of an active reagent-containing zone under the influence of vacuum-induced compaction.

## DESCRIPTION OF THE PREFERRED EMBODIMENTS

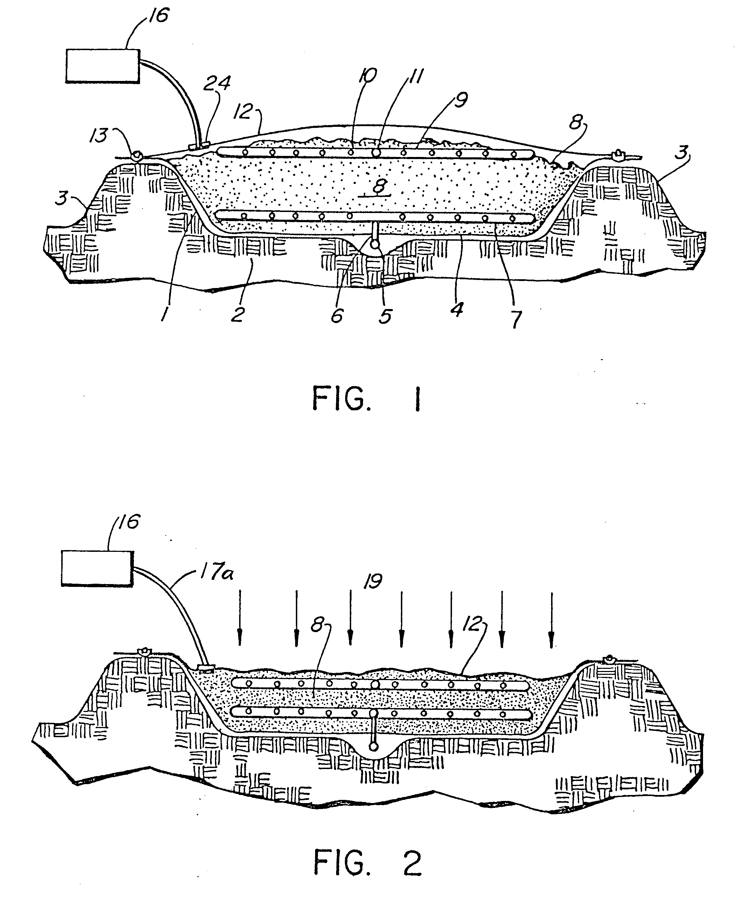

In Figure 1 a depression 1 in the earth 2 has been formed within an encircling berm 3. The surface of the depression 1 is lined with a lower sealing layer 4 which may be flexible plastic sheet 4 made from vinyl, polyethelene or a similarly compatible material. In a more complex embodiment the surface of the depression 1 may be sealed by cement or some other sealing product.

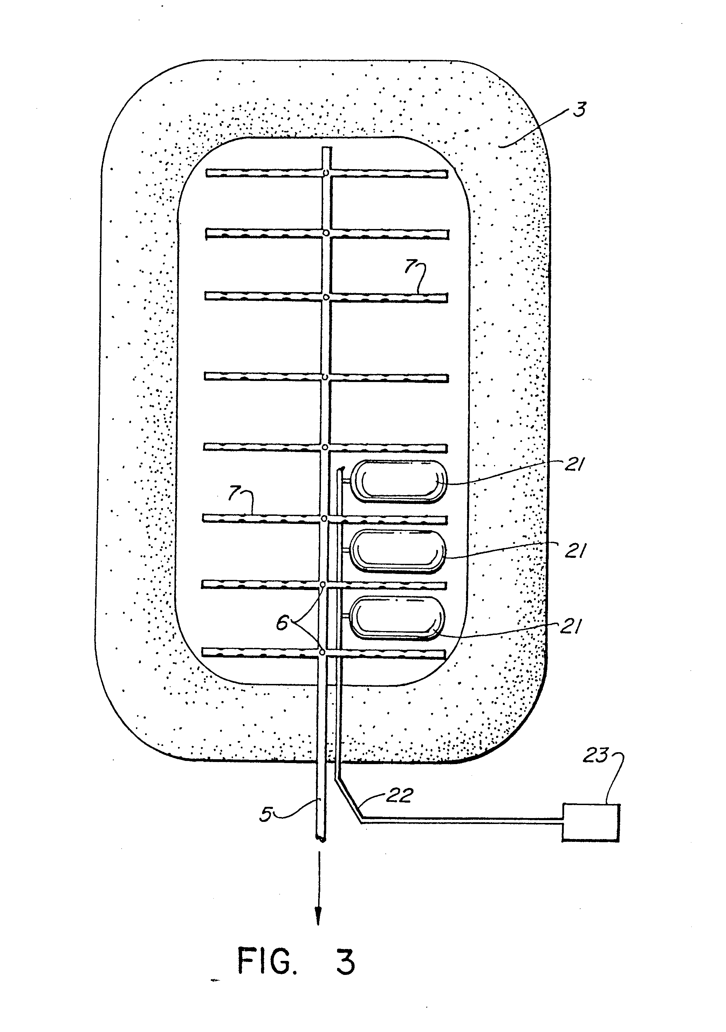

In a central region of the depression 1 a drainage pipe 5 is installed below the lower sealing layer 4. This pipe 5 communicates through a lower sealed coupling 6 to a network of fluid-extraction tubing 7, located on the other side of the sealing layer 4, that constitutes a collection system. This tubing 7, shown most clearly in Figure 3, may conveniently be made of perforated plastic tubing that is suitable for the collection of liquids.

Figure 3 shows an overhead view of the collection system within the container of Figure 1.

Between the tubing 7 may be installed on the floor of the depression 1, above the sealing layer 4, inflatable bladders 21. Only three such bladders are shown in Figure 3 for simplicity. In use, bladders 21 would be placed between all of the tubes 7. In one alternative, where the lower sealing layer 4 is a flexible sheet, the bladder 21 may be formed by bonding an upper flexible sheet to lower layer 4.

The array of bladders 21 are connected through their own piping network 22 to an external source 23 of gas or fluid that can be used to fill the bladders and create pulsations therein. When it is desired to agitate the biomass 8 the bladders 21 may be inflated and deflated repeatedly.

Returning to Figure 1, once the lower containment structure is complete biomass 8 is then deposited within the depression 1. A network of pipes 9 constituting the reagent distribution system 10 is then laid over the biomass 8. A central header 11 provides an inlet to distribution system 10 and communicates with the exterior region through sealed connectors (not shown) passing through the upper cover 12 in a manner that will preserve an air tight seal.

Over the distribution system 10 and biomass 8 is placed a collapsible upper cover 12 which may again be made of vinyl or polyethelene sheeting, or a similar suitable material. This upper cover 12 may also be a ridged panel, provided that it has flexible sides that may be sealed to the lower sealing layer 4 in a manner akin to that to be described for sheeting, or other means that allow its vertical movement while maintaining the air-tightness of the containment structure.

The sides of the upper cover 12 extend completely over the biomass and down to the border 12 of the lower sealing layer 4 where an air tight seal may be effected.

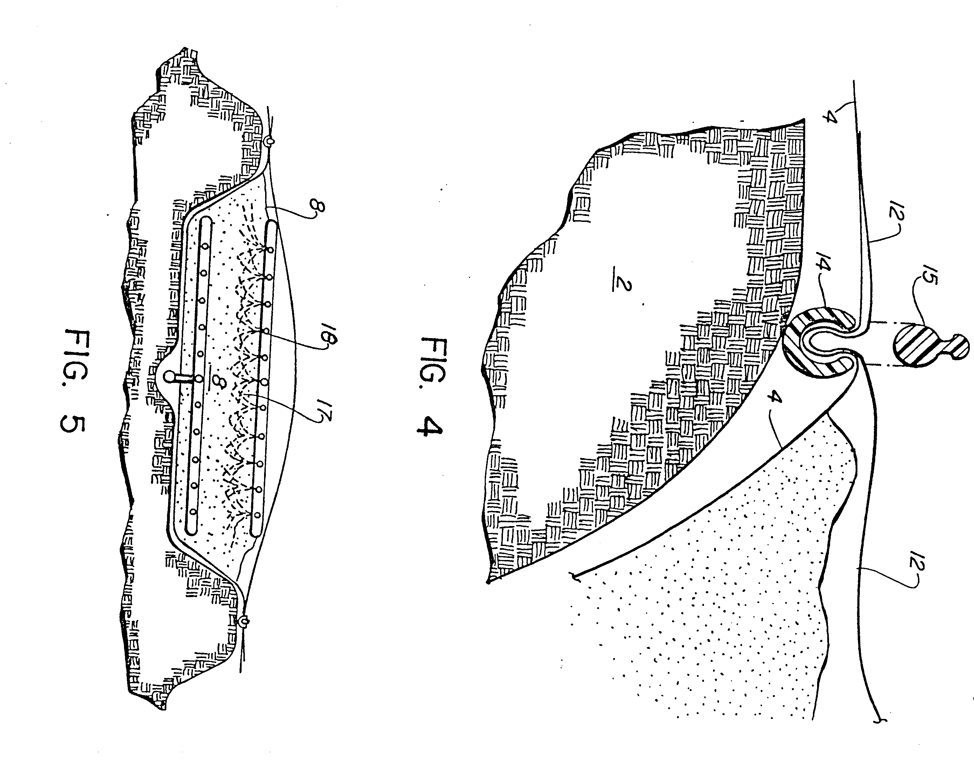

Figure 4 shows an enlarged view of one means by which the upper cover 12 may be attached to the lower sealing layer 4, in the case where the lower sealing layer 4 is composed of flexible sheeting.

On the surface of the berm 3 a "C" cross-section sealing strip 14 of flexible material, suitably of extruded plastic, is laid around the entire quantity of biomass. The lower sealing layer 4, in this case a plastic sheet, is laid over the sealing strip 14 so that it may be pressed into the "C" shaped channel. The upper cover 12 is then laid over the same sealing strip 14 and a linear plug 15 inserted snugly within the channel of the sealing strip 14 which is sprung to close in a clamping fashion. This arrangement will provide a sufficient seal to allow an adequate vacuum to be drawn.

A vacuum may be created by use of the standard vacuum pump 16, or any equivalent device for creating a reduced air pressure within the containment structure, shown schematically in Figures 1, 2 and 6. This pump may draw air through a hose 17a which communicates with the region under the upper cover 12 through a sealed coupling 24. Once the vacuum pump 16 is activated, the atmospheric pressure arising from the difference created by the reduced internal gas pressure shown by arrows 19 in Figures 3 and 5, will apply a compressive force to the upper cover 12. This will cause a collapse and compression of the biomass 8.

Although a "vacuum" is referred to this does not refer to a "perfect" or "high" vacuum. Rather, this word is intended to refer to a condition of reduced pressure sufficient to effect a compression of the biomass matrix.

The ability to effect a compression of the biomass is useful in these respects. If a measured quantity of reagent has been added to the biomass 8 through the distribution system 10, the biomass 8 may be compressed under vacuum to assist in the thorough wetting thereof. If the biomass has been through the digestion process the liquid products may be expressed from the biomass by this compression and withdrawn by the collection system tubing 7 after the liquids have accumulated at the bottom of the biomass 8 under gravity. Water may further be applied, as if it were a reagent, to flush out residual reagent for subsequent separation and recovery.

Again, the vacuum compression procedure can enhance the extraction of this rinse water.

When it is desired to remove fluids via the collection system tubing 7, the bladders 21 may be inflated fully to create sloping surfaces that will direct fluids under gravity to that tubing 7. In such application, bladders 21 that are bonded integrally to the lower layer 4 will provide superior performance.

The digestion process may be initiated before drawing a vacuum, by introducing the reagent through the distribution system 10. Sufficient reagent may be added to wet the entire amount of biomass after compression. Alternately only sufficient reagent may be introduced to wet just an upper zone. This depicted in Figure 5.

In Figure 5 reagent 18 is being deposited in the upper layer 17 of the biomass 8. Once this layer is wetted the digestion reaction is allowed to proceed to relative completion within the upper layer 17.

A highly concentrated form of reagent 18 may be applied at this stage, allowing that only a reduced quantity is needed to wet the limited volume of the upper layer. This will result in an increase in the rate of the reaction occurring in the upper layer.

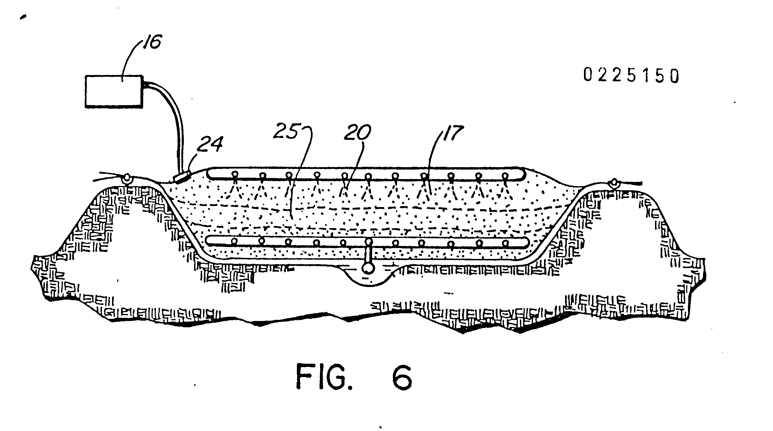

Then a displacing or diffusing fluid 20 is distributed over the upper layer 17 by the distribution system. This is shown in Figure 6. By choosing a displacing fluid 20 that does not impair the performance of the reagent, the presence of this fluid will serve to displace the reagent downward into a lower layer. Alternately, if the displacing fluid 20 dilutes the reagent, such as where water is used, then by using a highly concentrated initial deposit of reagent, the diluted reagent will still retain an adequate degree of reactivity. Thus this second flushing will serve to diffuse the reagent further within the biomass.

The former upper layer 17 will then become occupied by displacing fluid or reagent and displacing fluid combined, and the displacing fluid 20 and the reagent 18 will be carried into a lower zone 25. This process can be enhanced by compressing the biomass 8 under vacuum.

The new zone 25 of reagent 18 may then be allowed to react with the biomass 8 until it is digested. Then further displacing fluid 20 may be applied so as to move the reagent 18 downward to an even lower zone.

The advantage of this process is that less quantity of reagent may be required in order to process the initial quantity of biomass. If the initial application is of a concentrated form of reagent, the reaction will then proceed much faster. Thus, through use of this expanding zone reaction system, the length of time necessary for the entire quantity of biomass to be digested will be reduced.

In operation the sealed containment of the biomass effected by the invention allows the biomass to be held in isolation from exterior contamination by microbes and yeasts. Where it is decided to store biomass in a condition where it will not deteriorate by rotting, an acid reagent e.g. 3.0 pH, may be applied. This will preserve the biomass in a "pickled condition".

When it is desired to reactivate the digestion process, the acid may be flushed-out by applying a large quantity of water, or may be neutralized by applying a basic solution.

The foregoing disclosure constitutes a description of several preferred embodiments of the invention. The invention in its broadest and more particular aspects is more precisely defined in the claims which follow.

The presence of reagent distribution 10 and collection systems 7 within the enclosure for the biomass 8 has further particular utility. Heat generated within the biomass 8 may be removed by extracting liquids that carry the heat with them, cooling the liquids externally, and then reinjecting the liquids through the distribution system 10 where required to maintain the digestion process or to further cool the biomass.

The application of vacuum compression to the biomass 8 will facilitate this cooling cycle by speeding the expression of heat-removing liquids from the biomass.

The ability to recycle digestion fluids through the biomass after extraction can also serve to partially filter-out yeast organisms suspended in such liquids. Again, vacuum compression may be applied so as to compact the fiberous mass of biomass within the digester. In this compacted condition the digestion liquid may be repeatedly removed through the bottom collection system 7 and reapplied from above through the upper distribution system 10 where it may then percolate downwards under gravity This repeated cycling of the liquid through the compacted biomass will serve to partially filter-out yeast organisms.

## Claims

1. A containment structure for use in compacting a matrix of biomass comprising:
    (a) a compressible, enveloping air-tight enclosure having an upper cover and,
    (b) a vacuum pump communicating with said containment structure through a conduit adapted to evacuate gas from the containment structure.

2. A containment structure as in claim 1 wherein the upper cover of said containment structure comprises a flexible sheet which forms the upper surface of said structure and is adapted to be displaced downwardly into said structure upon development of a vacuum therein.

3. A containment structure as in claim 1 wherein the upper cover of said structure is displaceable downwards into said structure upon development of a vacuum therein, and wherein the outside border of said upper cover is comprised of flexible sheet material.

4. A containment structure as in claims 1, 2 or 3 including reagent-distribution and collection systems installed in the upper and lower regions within said containment structure.

5. A containment structure as in claims 1, 2 or 3 for containing biomass having:

(a) a fluid collection system comprised of a series of spaced collection tubes located along the floor of said structure and,

(b) inflatable bladders located along the floor of said structure in spaces between said collection tubes, said bladders being adapted to agitate said biomass upon inflation and deflation, and further being adapted on inflation to direct fluid falling within the containment structure under the influence of gravity towards said collection tubes.

6. A containment structure as in claim 5 wherein said bladder is formed by bonding an upper flexible sheet to the lower surface of the containment structure.

7. A method of wetting with reagent biomass located in a containment structure as in claim 4 comprising applying the requisite quantity of reagent through said reagent distribution system and then drawing a vacuum within the enveloping enclosure so as to thereby allow the atmospheric air pressure to compact the biomass, reduce the percentage of voids therein and increase the wetting of the biomass by the reagent.

8. A method of wetting with reagent, biomass located in a containment structure as in claim 5 comprising applying the requisite quantity of reagent through said reagent distribution system and then drawing a vacuum within the enveloping enclosure so as to thereby allow the atmospheric air pressure to compact the biomass, reduce the percentage of voids therein and increase the wetting of the biomass by the reagent.

9. A method of wetting with reagent biomass located within a containment structure comprising applying a limited quantity of reagent to the upper surface of the biomass, in a quantity insufficient to wet more than an upper layer of said biomass, allowing the digestion to proceed in said wetted upper layer, and subsequently, when the digestion process has been essentially exhausted in said upper layer, applying a displacing fluid to said biomass from above so as to cause the residual reagent in said biomass to be moved downward into a lower layer within the biomass, that has not been exhausted through digestion.

10. A method according to claim 9 wherein said containment structure is as in claim 4 and wherein a vacuum is drawn within said enveloping enclosure so as to allow the atmospheric pressure to compress the fiber matrix of the biomass after said displacing fluid has been applied thereto.

11. A method according to claim 9 wherein said containment structure is as in claim 5 and wherein a vacuum is drawn within said enveloping enclosure so as to allow the atmospheric pressure to compress the fiber matrix of the biomass after said displacing fluid has been applied thereto.

12. Compacted biomass produced by means of the structure or method of any of the preceding claims.

13. A method of facilitating the digestion of biomass within a containment structure comprising extracting from he the biomass liquids that have accumulated heat in the course of the digestion process, cooling such liquids externally to the containment structure, and reinjecting such fluids in a cooled condition into the biomass.

14. A method according to Claim 13, wherein the containment structure is as defined in Claims 4, 5 or 6.

15. A method of reducing the concentration of yeast organisms in liquids found within biomass that is undergoing digestion within a containment structure as defined in Claims 4, 5 or 6 comprising:

(a) Removing said liquid from said biomass through said collection system at the lower portion of said biomass,

(b) Applying a reduced air-pressure condition within said containment structure so as to compact said biomass,

(c) Re-inserting said liquid into the containment system through said distribution system at the upper portion of said biomass,

(d) Allowing said liquid to percolate under gravity through said compacted biomass: and

(e) Re-extracting said liquid through said collection system whereby the re-extracted liquid has a reduced concentration of yeast organisms therein.

FIG. 1

FIG. 2

0225150

FIG. 3

0225150

FIG. 4

FIG. 5

FIG. 6

0225150